# EUROPEAN PATENT APPLICATION

(11) **EP 1 790 770 A2**
(43) Date of publication of application: **30.05.2007**
(21) Application number: 06380308.4
(22) Date of filing: 22.11.2006
(51) Int. Cl.: D06M 10/02, D06M 23/16, D04H 3/00

(54) **Procedure for obtaining nonwoven fabrics with hydrophilic areas and hydrophobic areas**

(30) Priority: 23.11.2005 ES 200502891
(71) Applicant: Tesalca-99, S.A., 08006 Barcelona (ES)
(72) Inventor: Conillera, Trillas, Xavier Edificio Alfonso XII, 08006 Barcelona (ES); Garcia Pano, Robert Edificio Alfonso XII, 08006 Barcelona (ES); Guasch Riera, Rosa Edificio Alfonso XII, 08006 Barcelona (ES)
(74) Representative: Carpintero Lopez, Francisco

(57) **Abstract**

Provides a nonwoven fabric with totally or partially hydrophilic areas and hydrophobic areas having a specific geometry and, optionally, a hydrophilia gradient. The procedure for manufacturing said fabric comprises a stage of treatment with a plasma, either in a vacuum or at atmospheric pressure, in which controlling the plasma application and generation parameters allows controlling the specific area and number of hydrophilic groups incorporated in the surface of the fabric. Said fabric can be used in applications of the hygiene and health sector for manufacturing diapers, products for incontinence or women's hygiene products, for example.

## Description

### FIELD OF THE INVENTION

The present invention relates to the field of nonwoven fabrics manufactured from polyolephins, which by nature are hydrophobic, i.e. they tend to repel water. More specifically, it relates to a procedure for obtaining nonwoven fabrics with totally or partially hydrophilic areas and hydrophobic areas with a specific geometry and, optionally with a hydrophilic gradient. Said fabrics can be used as a top layer in various hygienic products as they have advantageous dryness and comfort properties.

### BACKGROUND OF THE INVENTION

Many applications of the hygienic sector require hydrophilic fabrics. Thus, in most currently manufactured diapers, incontinence products and women's hygiene products the top layer or top sheet that comes in contact with urine or other fluid is hydrophilic.

Currently, the treatment used to convert a nonwoven fabric from hydrophobic to hydrophilic involves surface treatments with hydrophilising agents or tensioactive solutions referred to as surfactants.

Surfactants are substances which from a physicochemical point of view have polar-nonpolar duality. The polar part shows affinity for polar solvents, in particular water, and is thus hydrophilic. On the other hand, the nonpolar group is the hydrophobic or lipophilic part.

When a nonwoven fabric is subjected to a treatment with surfactant solutions, once dried the fabric contains only the active part of the solution, the surfactant. In the case of a nonwoven fabric made with polypropylene, the hydrophobic part of the surfactant faces the polypropylene chains (100% nonpolar) and the hydrophilic part faces away from the polypropylene chains, increasing the critical surface tension of polypropylene and conferring hydrophilic properties to the nonwoven fabric.

When a drop of liquid comes in contact with a solid, several forces act on the drop: the surface tension of the liquid, the critical surface tension of the solid and the interface tension between the two compounds. The relation of these forces will determine the higher or lower resistance of the fabric to passage of water or other liquids.

Therefore, out of the three parameters, two (solid critical surface tension and interface tension between the fabric and the liquid) depend directly on the raw material.

The critical surface tension of polypropylene is approximately 29 mN/m, while that of pure water is 72 mN/m. The smaller this difference (such as by increasing the value of the critical surface tension of polypropylene) the more hydrophilic the material will be.

However, the use of solutions with liquid hydrophilising agents has certain drawbacks:
1. Significant generation of waste materials requiring treatment. This represents a high handling and processing cost.
2. Treating the nonwoven fabric with a solution requires subsequent drying of the water, and therefore energy expenditure.
3. Solutions cannot be stored and reused due to the risk of bacterial contamination, and the solution system must be thoroughly cleaned when changing from one agent to another. This implies an added cost and a risk of bacterial contamination.

An alternative technology for obtaining nonwoven fabrics with hydrophilic properties is plasma technology. Application of plasma technology on textiles began in Russia in the 1960's, gaining ground in the United States and Europe in the 1980's. Since then studies have been published with the results of experiments conducted in vacuum reactors designed mainly to process inorganic materials.

The advantages of these new plasma technologies with respect to the wet method are obvious:
1. Reduction or elimination of liquid hydrophilising agents and water.
2. Reduction or elimination of the waste material generated when using liquid hydrophilising agents.
3. Lower risk of bacterial contamination.

However, the hydrophilic finishes obtained are uniform.

The state of the art describes nonwoven fabrics with specific hydrophilic areas based on a number of openings made in their surface (US 6,911,573).

### OBJECT OF THE INVENTION

The object of the present invention is therefore to provide a nonwoven fabric with totally or partially hydrophilic areas and hydrophobic areas, with a specific geometry of the hydrophilic areas on its surface.

Another object of the invention is to provide a procedure for obtaining said nonwoven fabric with totally or partially hydrophilic areas and hydrophobic areas.

Lastly, another object of the invention is to provide the use of said nonwoven fabric with totally or partially hydrophilic areas and hydrophobic areas in applications of the hygiene or health sector.

### DESCRIPTION OF THE INVENTION

In one aspect of the invention, a nonwoven fabric is provided with totally or partially hydrophilic areas and hydrophobic areas with a specific geometry of the hydrophilic areas on its surface.

In a specific embodiment, the hydrophilic areas have a hydrophilic gradient.

In this way it is attempted to generate in the nonwoven fabric hydrophilic areas of different or like degree, so that in one edge of the area more liquid will pass that in the other. Absorbent and non-absorbent areas, as gradient areas, are not randomly located.

The geometry can be any according to the intended use of the nonwoven fabric. Thus, hydrophilic areas can be obtained in the top sheet of a diaper where liquid is meant to pass, and other untreated areas that will be hydrophobic.

In a specific embodiment said nonwoven fabric is made with a polyolephin. In a preferred embodiment, said nonwoven fabric is made with polypropylene. As mentioned above, polypropylene is one of many hydrophobic organic polymers used to manufacture hydrophobic nonwoven fabrics, that is, with a degree of water repellent properties.

In another specific embodiment said nonwoven fabric is a spunbound fabric. In another specific embodiment said nonwoven fabric is a meltblown fabric.

Spunbound fabrics are formed of continuous fibers of varying smoothness with a diameter on the order of 17-20 microns joined to one another by mangling. Thus, by generating nearby welding points joined by straight and comparatively strong fiber swaths the fiber will have a certain stiffness.

Meltblown fabrics are formed by continuous fibers on the order of 4 to 7 microns that are joined to one another on their own without requiring a final joining process (such as mangling or hydroligands).

In another aspect of the invention a procedure is given for obtaining a nonwoven fabric with totally or partially hydrophilic areas and hydrophobic areas as described above, comprising a stage in which the fabric is treated with a gaseous plasma.

In the context of the invention, the term "plasma" refers to a partially ionized gas consisting of ions, electrons and neutral species that can modify the surface of the substrate to be treated without modifying the intrinsic properties of said substrate.

The plasma is generated by applying a large amount of energy to a gas volume. The following parameters determine the final characteristics of the surface to be treated:
- Composition of the gas
- Plasma generation and application parameters: pressure, power, application time and application distance
- Substrate properties.

The final characteristics of the surface to be treated that can be obtained depending on the above three parameters are varied: hydrophilia, hydrophobia, printing and sterilization, among others. The object of this invention is to obtain hydrophilic properties to varying degrees in specific areas of the fabric.

As mentioned above, the fabric is a spunbond o meltblown nonwoven fabric, or a combination thereof, made of polyolephins in general and polypropylene in particular. In a specific embodiment, said fabric is made of polypropylene.

When polypropylene is bombarded with plasma a number of chemical and/or physical reactions take place that modify the hydrophobic nature of polypropylene.

Depending on the gas employed the reactive species such as ions present will be different and thus their action on polypropylene will lead to different final characteristics. The gas employed can be organic, inorganic or a liquid precursor. The modification of the substrate by plasma treatment is superficial and does not affect the intrinsic properties of polypropylene, that is, its mechanical properties, for example tractions and elongations.

There are different ways of inducing gas ionization depending on the technology applied:
- Glow discharge (at low pressures, vacuum plasma)
- Corona discharge (at atmospheric or slightly higher pressures)
- Dielectric barrier discharge (at atmospheric pressure).

The gas ionization source can be, for example, a system of two electrodes between which is applied the electric power of a radiofrequency generator.

The object of this invention is to obtain hydrophilic properties using vacuum plasma or atmospheric pressure plasma technologies. In the case of vacuum plasma a greater uniformity and flexibility is obtained than with any other plasma treatment.

The plasma is applied on a variable geometry by controlling certain plasma generation and application parameters. This allows controlling the area and number of hydrophilic groups incorporated to the surface of the nonwoven fabric.

The plasma generation and application parameters controlled are the following:
- Power or voltage of the gas ionization source used to generate the plasma;
- Frequency of the gas ionization source used to generate the gaseous plasma;
- Distance of application of the plasma; and
- Time of application of the plasma.

One or several of these parameters will be varied according to the degree and size of the hydrophilic areas desired.

Said parameters can be controlled, for example, by a computer using suitable software.

In addition, a plasma gradient on a spunbond or meltblown nonwoven fabric, for example, allows obtaining a greater or lesser deposition of hydrophilic groups. Their number will make the nonwoven fabric more or less hydrophilic in different areas according to the gradient used.

To control the area and number of hydrophilic groups incorporated to the surface of the nonwoven fabric, application of the plasma by areas is foreseen. There are different options for applying plasma by areas.

In a first embodiment the plasma is projected from several individual nozzles, each of which projects on a specific area defining a specific geometry in the nonwoven fabric. These nozzles have an on/off frequency controller and allow obtaining partially hydrophilic areas.

In a second embodiment the plasma is projected from a single nozzle placed along the width of the nonwoven fabric, while between said nozzle and the substrate to be treated is placed a sheet of stainless steel, aluminum or another material provided with orifices. These orifices can have different geometries and can be distributed in the surface of the sheet in very different manners, so that the nonwoven fabric obtained will have hydrophilic areas with a geometry defined by the plasma projection passing through the orifices of the sheet.

A third embodiment comprises a single nozzles placed along the entire width of the fabric with software that allows defining the number of plasma pulses per unit time. This allows applying the plasma intermittently on the nonwoven fabric that moves under the nozzle, obtaining previously defined hydrophilic and hydrophobic areas in the final product.

Another group of solutions are centered on a treatment after the plasma treatment, consisting of a vaporized monomer deposition with hydrophilic groups. This confers lasting properties as the anchoring of the functional groups is much stronger.

The use of the plasma as pretreatment implies the activation of the nonwoven fabric fibers for a subsequent coating treatment by vaporized monomer deposition with hydrophilic functional groups. This deposition is made in the areas of the nonwoven fabric previously treated with the plasma. It is necessary to provide a vacuum box in the system under the nonwoven fabric to direct and collect the vapor generated. After vaporization the monomer must set to obtain a uniform treatment of the treated areas.

The deposition treatment can be performed using a monomer evaporator and a plate with orifices placed along the width of the nonwoven fabric. Said orifices can have different geometries and be distributed in the surface of the plate in many different manners.

In another possible embodiment the deposition treatment is performed using several evaporators through corresponding individual rotating cylindrical plates, with orifices and orientated towards the nonwoven fabric. Said orifices can have different geometries and be distributed in the surface of the plate in many different manners.

In either case the nonwoven fabric obtained will have hydrophilic coated areas and hydrophobic areas at previously defined places distributed with a specific geometry.

In another aspect of the invention the use of said nonwoven fabric with totally or partially hydrophilic areas and hydrophobic areas is provided for applications of the hygiene or health sector. In a preferred embodiment, said fabric is used to manufacture a hygiene or sanitary product such as a diaper, a product for incontinence, a woman's hygiene product or an absorbent product for use in surgery. Said product is much more comfortable as the procedure of the invention allows a topsheet design that leaves the skin in contact with it drier and cleaner. In this way, the hydrophilic areas of the topsheet of the hygienic product carry bodily fluids towards the absorbent core, providing a sensation of dryness and comfort, keeping the skin from being wet too long and thereby preventing problems related to excessive skin hydration or contact with biological or chemical materials in the fluids.

### BRIEF DESCRIPTION OF THE FIGURES

To complete the description being made and in order to aid a better understanding of the characteristics of the invention, according to an example of a preferred embodiment, a set of drawings is accompanied forming an integral part of the invention where for purposes of illustration and in a nonlimiting sense the following is shown:
Figure 1 shows a schematic representation of a plasma projection system through individual nozzles applied on a nonwoven fabric.
Figure 2 shows a schematic representation of a plasma projection system through a single nozzle applied along the width of the fabric with an interposed sheet with orifices.
Figure 3 shows a schematic representation of a plasma projection system through a single nozzle along the width of the fabric, with software for defining the number of pulses which translate into plasma projections per unit time.
Figure 4 shows a schematic representation of a monomer deposition coating system through a plate, as well as a detailed view of the uneven structure obtained in the nonwoven fabric.
Figure 5 shows a possible monomer deposition coating through monomer evaporators with plates with orifices oriented towards the nonwoven fabric, as well as a detailed view of the uneven structure obtained in the nonwoven fabric.
Figure 6 shows another detailed view of an uneven structure of the fabric obtained with the procedure of projection by areas.
Figure 7 shows a detailed view of the uneven structure of a diaper obtained with the procedure of projection by areas.

### PREFERRED EMBODIMENT OF THE INVENTION

A preferred embodiment of the invention is described below with reference to the figures.

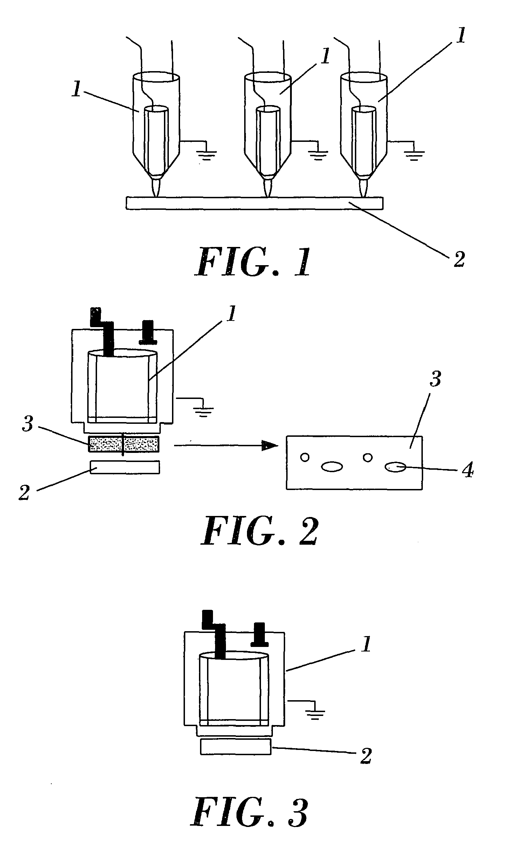

As seen in figures 1 and 3, the procedure for obtaining nonwoven fabric with totally or partially hydrophilic areas and hydrophobic areas that constitutes the object of this invention mainly consists of the partial projection of plasma on certain areas of the surface of a nonwoven fabric (2) manufactured from a hydrophobic material, preferably polyolephin, for obtaining in these sectors a geometry of hydrophilic areas (5) and/or partially hydrophilic areas (7) between hydrophobic areas (6).

Figures 1 to 3 show various options regarding the means used to apply the plasma.

Figure 1 shows several nozzles (1) that project plasma on a nonwoven fabric (2) in different sectors while moving continuously.

Figure 2 shows the nozzle (1) projecting plasma on the entire width of the nonwoven fabric (2) through a sheet (3) provided with orifices (4) in order to define hydrophilic areas (5) in the nonwoven fabric in correspondence with said orifices (4).

Figure 3 shows the nonwoven fabric (2) that moves under a nozzle (1) which projects plasma at different times controlled by software.

In addition, the nozzles (1) can move to define different geometries of hydrophilic areas (5) or partially hydrophilic areas (7) on the nonwoven fabric (2).

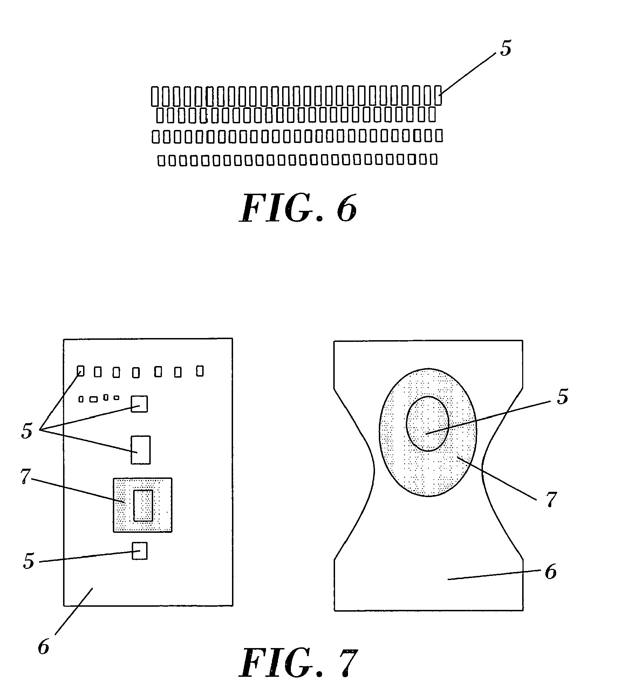

The result of the uneven application of the plasma projection on the nonwoven fabric (2) creates a specific geometry as shown in figure 6, or as in the case of figure 7 which shows a diaper with hydrophilic areas (5), hydrophobic areas (6) or partially hydrophilic areas (7).

The nozzles (1) incorporate frequency counters (not shown) that allow changing the plasma projection frequency to obtain partially hydrophilic areas (7) on the nonwoven fabric (2).

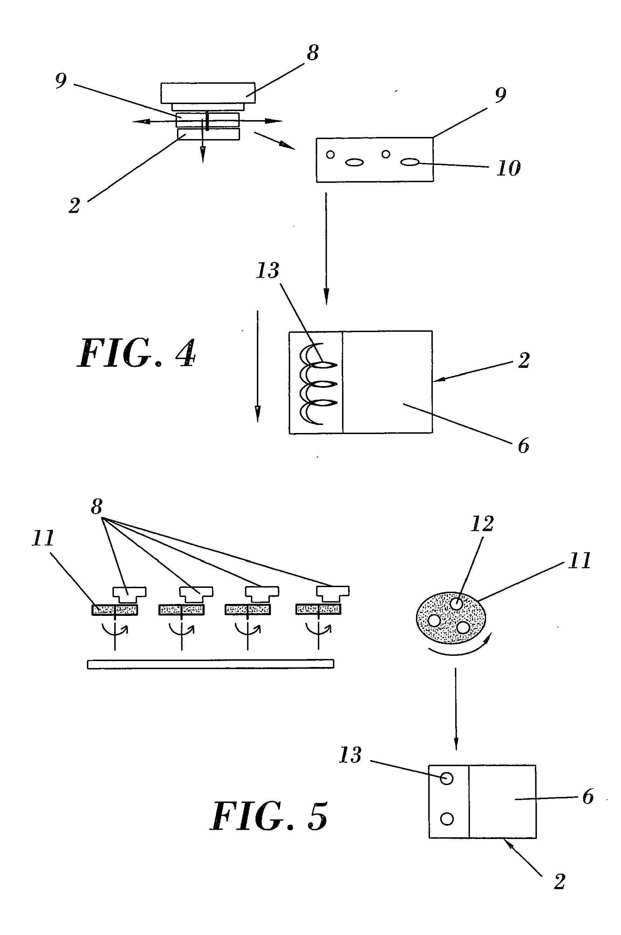

Figure 4 shows a monomer evaporator (8) applied in addition to the plasma projection on the hydrophilic areas (5) of the nonwoven fabric (2) to produce coated hydrophilic areas (13). Figure 4 also shows a plate (9) with openings (10) through which the monomer passes to coat the hydrophilic areas (5).

In addition, figure 5 shows several monomer evaporators (8) in correspondence with which are placed rotating plates (11) provided with openings (12) through which the monomer is projected for deposition on the hydrophilic areas (5) of the nonwoven fabric (2), producing coated hydrophilic areas (13).

## Claims

1. Procedure for obtaining a nonwoven fabric with totally or partially hydrophilic areas and hydrophobic areas **characterized in that** it consists of a partial plasma projection on certain sectors of the surface of a nonwoven fabric (2) made of a hydrophobic material, preferably olephin, to obtain in said sectors a geometry of hydrophilic areas (5) and/or partially hydrophilic areas (7) between hydrophobic areas (6).

2. Procedure for obtaining a nonwoven fabric with totally or partially hydrophilic areas and hydrophobic areas according to claim 1 **characterized in that** the plasma projection is performed in a vacuum.

3. Procedure for obtaining a nonwoven fabric with totally or partially hydrophilic areas and hydrophobic areas according to claim 1 **characterized in that** the plasma projection is performed at atmospheric pressure.

4. Procedure for obtaining a nonwoven fabric with totally or partially hydrophilic areas and hydrophobic areas according to claims 1 and 2 **characterized in that** after the plasma is applied, on the hydrophilic area (5) or partially hydrophilic area (7) a coating is applied by deposition of a vaporized monomer with hydrophilic functional groups.

5. Procedure for obtaining a nonwoven fabric with totally or partially hydrophilic areas and hydrophobic areas according to claim 4 **characterized in that** after the coating applied by monomer deposition a setting stage is performed.

6. Procedure for obtaining a nonwoven fabric with totally or partially hydrophilic areas and hydrophobic areas according to claim 1 **characterized in that** the nonwoven fabric is polypropylene.

7. Procedure for obtaining a nonwoven fabric with totally or partially hydrophilic areas and hydrophobic areas according to claim 1 **characterized in that** the nonwoven fabric is a meltblown fabric.

8. Procedure for obtaining a nonwoven fabric with totally or partially hydrophilic areas and hydrophobic areas according to claim 1 **characterized in that** the nonwoven fabric is a spunbond fabric.

9. Procedure for obtaining a nonwoven fabric with totally or partially hydrophilic areas and hydrophobic areas according to claim 1 **characterized in that** the nonwoven fabric is a combination of spunbond and meltblown fabrics.

10. Nonwoven fabric obtained with totally or partially hydrophilic areas and hydrophobic areas according to the procedure described in claims 1 to 9.

11. Use of a nonwoven fabric with totally or partially hydrophilic areas and hydrophobic areas according to claim 10 in applications of the hygiene or health sector.

12. Use according to claim 11 for manufacturing diapers, products for incontinence or products for women's hygiene.

13. Use according to claim 11 for manufacturing absorbent products for use in surgery.
